# EUROPEAN PATENT APPLICATION

(11) **EP 4 411 374 A1**
(43) Date of publication of application: **07.08.2024**
(21) Application number: 22883817.3
(22) Date of filing: 30.09.2022
(51) Int. Cl.: G01N 33/50, G01N 33/493, A61B 10/00, G01N 21/78, B01L 3/00

(54) **KIT FOR URINE TESTING**

(30) Priority: 19.10.2021 KR 20210139593
(71) Applicant: DNC Biotechnology Inc., Daejeon 34134 (KR)
(72) Inventor: PARK, Seong su, Anseong-si Gyeonggi-do 17577 (KR)
(74) Representative: Manitz Finsterwald Patent- und Rechtsanwaltspartnerschaft mbB
(86) International application number: PCT/KR2022/014723
(87) International publication number: WO 2023/068593

(57) **Abstract**

A urine test kit includes a container configured to contain urine; a paper strip guide including a groove and a paper strip which is formed in the groove and whose color is changed by the urine; and an air pump configured to enable the urine to flow to the paper strip. Wherein the paper strip guide is attached to one surface of the container to form a flow path between the groove in the paper strip guide and the one surface of the container.

## Description

### TECHNICAL FIELD

The present disclosure relates to a urine test kit.

### BACKGROUND

In general, a urine test is an essential examination that occupies 30% of the total number of examinations performed in most hospitals or clinics. The urine test is a kind of diagnostic method to diagnose systemic conditions and lesions by analyzing urine substances.

A conventional urine test is performed to examine urine by bringing a test stick with a paper test strip into contact with an examinee's urine and checking a color change of the paper test strip. That is, an examiner performs the urine test by directly applying the examinee's urine to the test stick, or the urine test is performed by collecting urine in a container such as a paper cup and bringing the test stick into contact with the collected urine.

However, during the conventional urine test, the examiner and the examinee may be exposed to secondary infection by the urine specimen, or the examiner is inconvenienced in that he/she has to directly apply the examinee's urine to the urine test strip.

Also, the accuracy in test is degraded due to different amounts of time from urine collection to urine test.

In this regard, Korean Patent No. 10-1904037, which is prior art, relates to a urine test kit and device in which urine collected into a container is pulled up along a urine flow path by capillarity caused by the urine flow path and guided to a test strip. Therefore, it is possible to check a color change of the test strip as soon as urine is collected. However, when it takes a long time from urine collection to urine test, the color of the test strip is changed differently from the color right after the urine test, and, thus, an accurate urine test cannot be accomplished. Therefore, an examiner is inconvenienced in that he/she has to perform a urine test right after urine collection.

### PRIOR ART DOCUMENT

(Patent Document 1) Korean Patent No. 10-1904037 (registered on September 27, 2018)

### DISCLOSURE OF THE INVENTION

### PROBLEMS TO BE SOLVED BY THE INVENTION

The present disclosure is conceived to provide a urine test kit with which an examiner can perform a urine test at any desired time after urine collection.

However, the problems to be solved by the present disclosure are not limited to the above-described problems. There may be other problems to be solved by the present disclosure.

### MEANS FOR SOLVING THE PROBLEMS

According to an aspect of the present disclosure, a urine test kit includes a container configured to contain urine; a paper strip guide including a groove and a paper strip which is formed in the groove and whose color is changed by the urine; and an air pump configured to enable the urine to flow to the paper strip. Wherein the paper strip guide is attached to one surface of the container to form a flow path between the groove in the paper strip guide and the one surface of the container.

According to another aspect of the present disclosure, a urine test kit includes a container configured to contain a liquid specimen; a sheet which is attached to one surface of the container and in which a liquid specimen flow path is formed and at least one paper strip whose color is changed by the liquid specimen is provided in at least a part of the flow path; and a pressing member configured to enable the liquid specimen to flow to the paper strip.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to any one of the above-described means for solving the problems of the present disclosure, it is possible to guide urine from the outside to a paper test strip by using an air pump. Therefore, an examiner can perform a urine test at any desired time after urine collection, and the reliability of the urine test can be improved.

### BRIEF DESCRIPTION OF THE DRAWINGS

**FIG. 1** illustrates a urine test kit according to an embodiment of the present disclosure.
**FIG. 2** illustrates a paper strip guide connected to an air pump according to an embodiment of the present disclosure.
**FIG. 3** shows another view of the urine test kit of **FIG. 1****.**
**FIG. 4** is an exemplary diagram illustrating that a connection hose of a urine test kit passes through a container connection hole according to another embodiment of the present disclosure.
**FIG. 5A** illustrates the status before pumping of the air pump in the urine test kit according to an embodiment of the present disclosure.
**FIG. 5B** illustrates the status after pumping of the air pump in the urine test kit according to an embodiment of the present disclosure.
**FIG. 6A** is a schematic diagram for explaining various arrangements of grooves formed in the paper strip guide according to an embodiment of the present disclosure.
**FIG. 6B** is a schematic diagram for explaining various arrangements of grooves formed in the paper strip guide according to an embodiment of the present disclosure.
**FIG. 6C** is a schematic diagram for explaining various arrangements of grooves formed in the paper strip guide according to an embodiment of the present disclosure.
**FIG. 6D** is a schematic diagram for explaining various arrangements of grooves formed in the paper strip guide according to an embodiment of the present disclosure.
**FIG. 6E** is a schematic diagram for explaining various arrangements of grooves formed in the paper strip guide according to an embodiment of the present disclosure.
**FIG. 6F** is a schematic diagram for explaining various arrangements of grooves formed in the paper strip guide according to an embodiment of the present disclosure.
**FIG. 6G** is a schematic diagram for explaining various arrangements of grooves formed in the paper strip guide according to an embodiment of the present disclosure.

### BEST MODE FOR CARRYING OUT THE INVENTION

Hereinafter, embodiments of the present disclosure will be described in detail with reference to the accompanying drawings so that the present disclosure may be readily implemented by a person with ordinary skill in the art. However, it is to be noted that the present disclosure is not limited to the embodiments but can be embodied in various other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Additionally, when a part "includes" one element, this means that it may further include another element rather than excluding another element, unless specifically stated to the contrary.

Hereinafter, the present disclosure will be explained in detail with reference to the accompanying configuration views or process flowcharts.

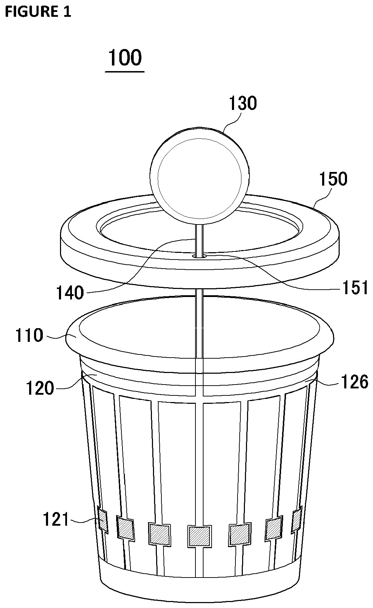

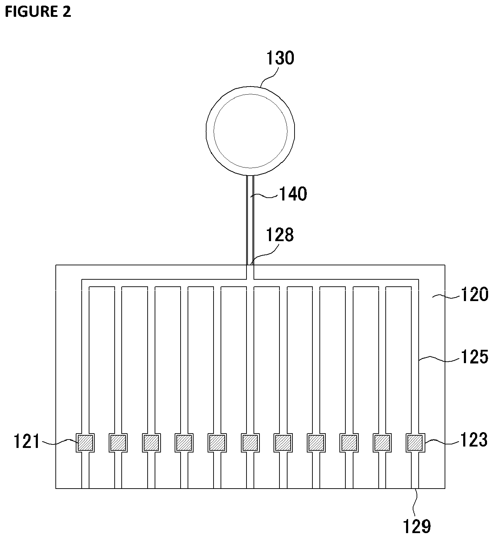

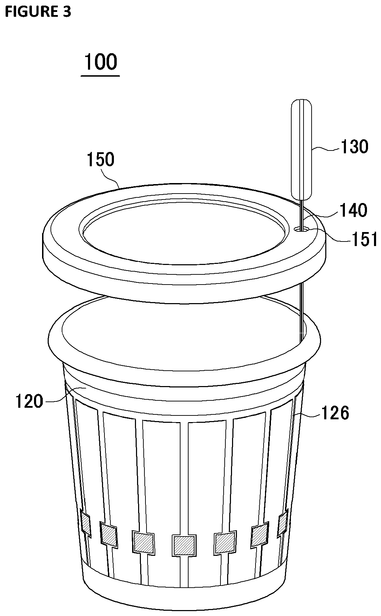

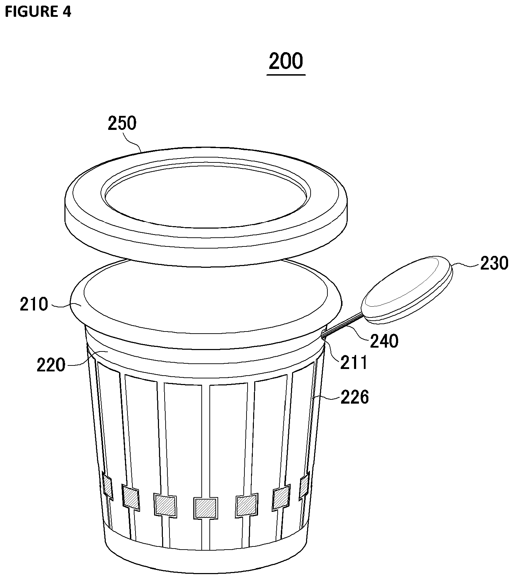

**FIG. 1** illustrates a urine test kit according to an embodiment of the present disclosure. **FIG. 2** illustrates a paper strip guide connected to an air pump according to an embodiment of the present disclosure. **FIG. 3** shows another view of the urine test kit of **FIG. 1****.** **FIG. 4** is an exemplary diagram illustrating that a connection hose of a urine test kit passes through a container connection hole according to another embodiment of the present disclosure.

First, referring to **FIG. 1****,** a urine test kit 100 according to an embodiment of the present disclosure may include a container 110, a paper strip guide 120, and an air pump 130.

The container 110 may be filled with urine, but is not limited thereto. The container 110 may be filled with various kinds of liquid specimens which can change the color of a paper strip 121.

Herein, the paper strip 121 may be a paper test strip whose color is changed by a liquid specimen such as urine. In an embodiment, the paper strip 121 may be implemented by litmus paper whose color is changed by urine, and a user can check the status of urine with naked eyes through the discolored litmus paper. For example, the paper strip 121 may be a paper test strip capable of screening diseases associated with occult bleeding, proteinuria, glucose, etc. through discoloration of urine.

At least a part of the container 110 may be formed of a transparent or semitransparent material. For example, the container 110 may be formed of a transparent plastic material. Thus, the user can check a residual quantity of urine in the container 110.

Further, the container 110 is illustrated as having a hollow cylindrical structure whose upper outer circumferential surface has a greater diameter than a lower outer circumferential surface, but may have a cylindrical structure whose upper outer circumferential surface has the same diameter as a lower outer circumferential surface. However, the present disclosure is not limited thereto. The upper outer circumferential surface or lower outer circumferential surface of the container 110 may have various diameter shapes, such as polygonal shapes including a square shape and pentagonal shape.

Referring to **FIG. 2****,** the paper strip guide 120 may include a groove 125 and the paper strip 121 formed in the groove 125. Specifically, the groove 125 is formed in the paper strip guide 120, and may include a paper strip insertion hole 123 into which the paper strip 121 can be inserted.

Herein, a plurality of grooves 125 may be formed in a lower portion of the paper strip guide 120 and merged into one groove in an upper portion of the paper strip guide 120. Therefore, the paper strip insertion hole 123 may be formed in each of the plurality of grooves 125 in the lower portion of the paper strip guide 120.

As described above, the paper strip 121 may be a paper test strip capable of screening diseases associated with occult bleeding, proteinuria, glucose, etc. through discoloration of urine. Therefore, in the plurality of grooves formed in the lower portion of the paper strip guide 120, the paper strips 121 required to test respective items may be sequentially arranged and inserted into the paper strip insertion holes 123.

Further, the paper strip 121 may be spaced apart by a predetermined distance from a lower end of the lower portion of the paper strip guide 120. In this case, the paper strip insertion hole 123 may be spaced apart by a predetermined distance from a lower end of each of the plurality of grooves 125 formed in the lower portion of the paper strip guide 120, and the paper strip 121 may be inserted into the paper strip insertion hole 123.

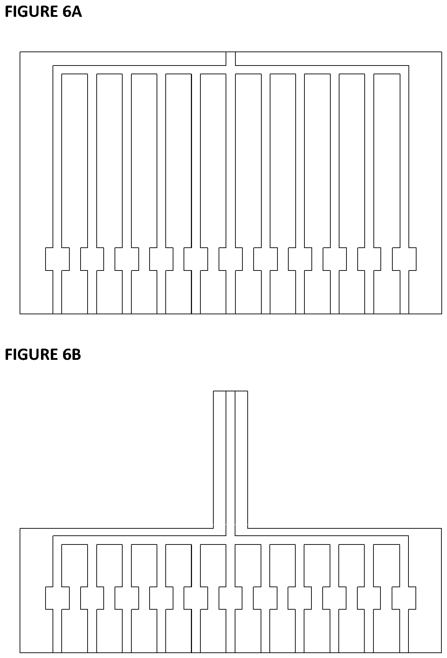

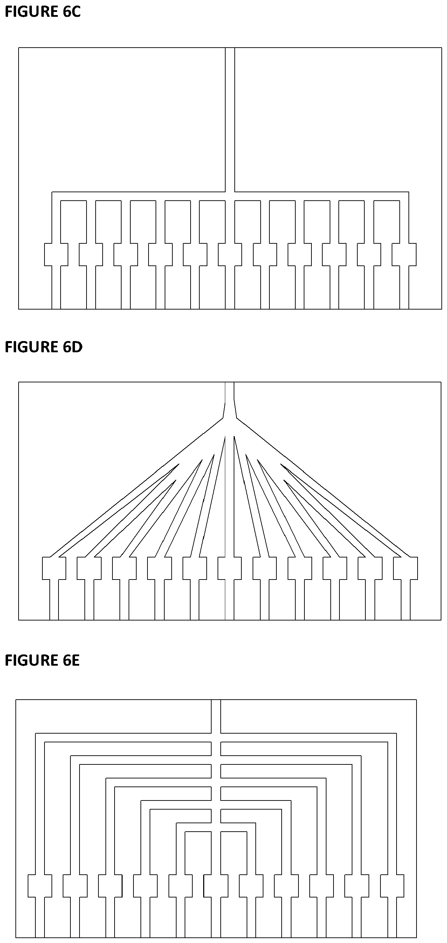

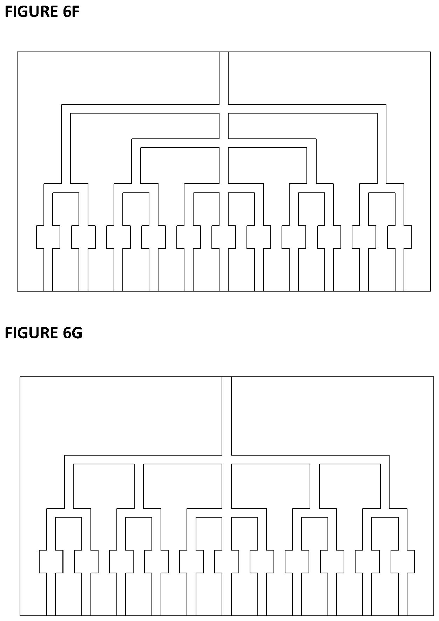

The arrangement of the grooves 125 formed in the paper strip guide 120 may be modified in various ways. This will be described with reference to **FIG. 6****.**

Referring back to **FIG. 1****,** the paper strip guide 120 may be attached to one surface of the container 110 to form a flow path 126 between the grooves 125 in the paper strip guide 120 and the one surface of the container 110. That is, the paper strip guide 120 may be implemented by a sheet which is attached to one surface of the container and in which a liquid specimen flow path is formed and at least one paper strip whose color is changed by a liquid specimen is provided in at least a part of the flow path.

Specifically, when the paper strip guide 120 of **FIG. 2** is attached along an inner circumferential surface of the container 110, the flow path 126 may be formed between the grooves 125 in the paper strip guide 120 and one surface of the container 110.

In an embodiment, the paper strip guide 120 may be spaced apart by a predetermined distance from a lower portion of the container 110 and attached along the inner circumferential surface of the container 110. Herein, the paper strip guide 120 may be spaced apart by a predetermined distance in order not to interfere with inflow of urine 10 in the container 110 into a lower end 129 of a lower flow path of the paper strip guide 120. Therefore, since the paper strip guide 120 is spaced apart by a predetermined distance from the lower portion of the container 110, the urine 10 in the container 110 can stably flow into the paper strip guide 120 through the flow path 126 formed in the paper strip guide 120.

The flow path 126 formed by attaching the paper strip guide 120 to the one surface of the container 110 may include the lower flow path and an upper flow path. The lower flow path may be formed in the lower portion of the paper strip guide 120 along the plurality of grooves, and the upper flow path may be formed in the upper portion of the paper strip guide 120 along the groove merged from the plurality of grooves.

The paper strip guide 120 may be connected to the air pump 130 in order for urine to flow to the paper strip 121. Herein, the air pump 130 may be formed of an elastic material to be deformed and configured to contain air therein. The air pump 130 may have a circular shape as shown in **FIG. 1** and **FIG. 2****,** but is not limited thereto. The air pump 130 may have various shapes, such as polygonal shape, a heart shape, a star shape, and the like.

The air pump 130 may be configured to enable the urine 10 to flow to the paper strip 121 through the flow path 126 of the paper strip guide 120 when a pressure difference is made between the inside and the outside of the air pump 130 through a pumping operation by compressing and decompressing the air pump 130. That is, as the volume the air pump 130 increases, the pressure decreases, and, thus, a liquid is introduced from the atmospheric pressure side according to Boyle's law. Therefore, the user can check the paper strip 121 whose color is changed by urine through a pumping operation by compressing the air pump 130.

The amount of urine introduced by the compression of the air pump 130 may be determined by the volume of the air pump 130 and a change in volume caused by pressure. Therefore, the air pump 130 may have various volumes or may be formed of various elastic materials to enable sufficient reaction of the paper strip 121 with the amount of urine which flows by one time compression.

In an embodiment, the air pump 130 may be configured as a pressing member which enables a liquid specimen to flow to the paper strip 121. When the pressing member is compressed, air inside the pressing member may be discharged to the outside, and when the pressing member is decompressed, the liquid specimen may flow to the paper strip 121 through the flow path 126 due to a pressure difference between the inside and the outside of the pressing member.

Meanwhile, the air pump 130 and the paper strip guide 120 are illustrated as being integrally formed with each other through a connection hose 140. However, this is just an example. In some embodiments, the air pump 130 and the paper strip guide 120 may be coupled to each other in various ways. Herein, the connection hose 140 may be connected to the air pump 130 and may serve as a passageway to transfer air.

For example, an upper end 128 of an upper flow path of the paper strip guide 120 may be coupled to a lower end of the connection hose 140 to communicate with the inside of the connection hose 140. For example, the connection hose 140 may be inserted and fitted into the upper flow path of the paper strip guide 120. Alternatively, the connection hose 140 may be attached and coupled to the upper end 128 of the upper flow path of the paper strip guide 120. Otherwise, the connection hose 140 may be integrally formed with the upper end 128 of the upper flow path of the paper strip guide 120.

Also, a lower end of the air pump 130 may be coupled to an upper end of the connection hose 140 to communicate with the inside of the connection hose 140. For example, the lower end of the air pump 130 may be fixed by being integrally formed with the connection hose 140 or may be detachably coupled to the connection hose 140.

As described above, since the air pump 130 is connected to the paper strip guide 120, the inside of the flow path 126 formed in the paper strip guide 120 may be sealed. Therefore, when no pressure is applied to the air pump 130, it is possible to suppress the flow of urine into the flow path 126 even if the container 110 contains urine therein. However, an adhesive force is generated by capillarity, and, thus, urine in the container 110 may be pulled up to a predetermined height along a wall of the flow path 126.

Therefore, as described above, the paper strip 121 may be spaced apart by a predetermined height from the lower end 129 of the lower flow path of the paper strip guide 120. Herein, the paper strip 121 may be spaced apart by a predetermined height from the lower end 129 of the lower flow path of the paper strip guide 120 so that even when urine contained in the container 110 is introduced to a predetermined height by capillarity through a lower end of the flow path 126 of the paper strip guide 120, it cannot be pulled up to the same height as the paper strip 121.

That is, since the paper strip 121 is spaced apart by a predetermined distance from the lower end 129 of the lower flow path of the paper strip guide 120, it is possible to suppress contact of the paper strip 121 with urine even when urine contained in the container 110 is pulled up to a predetermined height by capillarity through the flow path 126 formed in the paper strip guide 120.

Therefore, as the volume inside the air pump 130 is decreased or increased by compressing or decompressing the air pump 130, the pressure may be changed inside the connection hose 140 and the flow path 126 of the paper strip guide 120, and, thus, the urine may be introduced and discharged. Herein, preferably, the air pump 130 may be formed of a soft material to be elastically deformed upon compression by the user, but is not limited thereto.

If the air pump 130 formed of an elastic material is contracted by external pressure, air inside the air pump 130 may pass through the inside of the connection hose 140 and move to the outside through the flow path 126 of the paper strip guide 120.

A lid 150 may be detachably coupled to an upper portion of the container 110 to open or seal the container 110. The lid 150 is illustrated as being fitted into the upper portion of the container 110. However, this is just an example. In some embodiments, the lid 150 may be coupled to the container 110 in various ways. For example, the lid 150 may be screw-coupled to the container 110.

Herein, as shown in **FIG. 1** and **FIG. 3****,** the lid 150 may include a lid connection hole 151 in its upper surface, and, thus, the connection hose 140 which connects the air pump 130 and the paper strip guide 120 may pass through the lid connection hole 151. Specifically, the paper strip guide 120 connected to the air pump 130 through the connection hose 140 is attached along the inner circumferential surface of the container 110 and the lid connection hole 151 is formed in the upper surface of the lid 150, and, thus, the connection hose 140 may be implemented as passing through the lid connection hole 151.

In another embodiment, a urine test container 200 includes a container connection hole 211 in a side surface of a container 210, and, thus, a connection hose 240 which connects an air pump 230 and a paper strip guide 220 may pass through the container connection hole 211 as shown in **FIG. 4****.**

Specifically, the paper strip guide 220 connected to the air pump 230 through the connection hose 240 is attached along an inner circumferential surface of the container 120 to form a flow path 226. Also, the container connection hole 211 is formed in the side surface of the container 210, and, thus, the connection hose 240 may be implemented as passing through the container connection hole 211. Further, a lid 250 may be detachably coupled to an upper portion of the container 210 to open or seal the container 210.

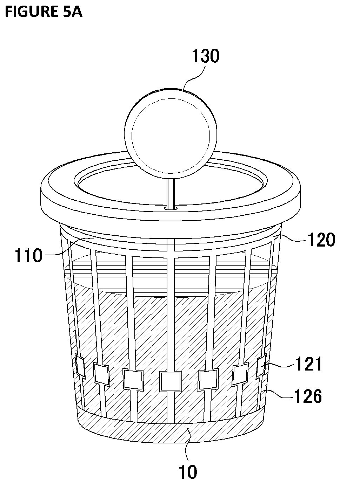

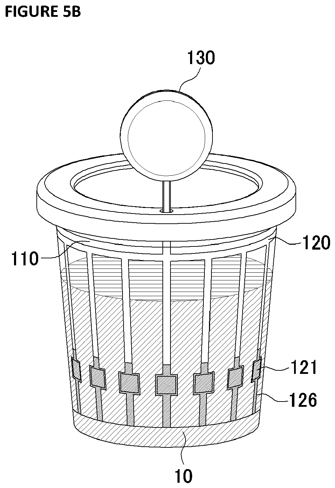

**FIG. 5A** illustrates the status before pumping of the air pump 130 in the urine test kit according to an embodiment of the present disclosure, and **FIG. 5B** illustrates the status after pumping of the air pump 130 in the urine test kit according to an embodiment of the present disclosure.

In **FIG. 5A** and **FIG. 5B****,** the paper strip guide 120 is illustrated as being transparent to show the flow of the urine 10 contained in the container 110 to the paper strip 121 through the flow path 126 of the paper strip guide 120.

First, as shown in **FIG. 5A****,** the air pump 130 is connected to the paper strip guide 120, and, thus, the inside of the flow path 126 formed in the paper strip guide 120 may be sealed. Therefore, when no pressure is applied to the air pump 130, it is possible to suppress the flow of urine into the flow path 126 even if the container 110 contains urine therein.

However, although not illustrated in the drawings, an adhesive force is generated by capillarity, and, thus, urine in the container 110 may be pulled up to a predetermined height along the wall of the flow path 126. Therefore, the paper strip 121 may be spaced apart by a predetermined height from the lower end of the lower flow path of the paper strip guide 120. Herein, the paper strip 121 may be spaced apart by a predetermined height from the lower end of the lower flow path of the paper strip guide 120 so that even when urine contained in the container 110 is introduced to a predetermined height by capillarity through the lower end of the flow path 126 of the paper strip guide 120, it cannot be pulled up to the same height as the paper strip 121.

That is, since the paper strip 121 is spaced apart by a predetermined distance from the lower end of the lower flow path of the paper strip guide 120, it is possible to suppress contact of the paper strip 121 with urine even when urine contained in the container 110 is pulled up to a predetermined height by capillarity through the flow path 126 formed in the paper strip guide 120.

Meanwhile, referring to **FIG. 5B****,** the urine 10 may be brought into contact with the lower end of the lower flow path of the paper strip guide 120 while the air pump 130 is compressed, and then, external pressure may be removed. In this case, the inside of the flow path 126 of the paper strip guide 120 and the inside of the air pump 130 may have a lower pressure than the outside.

Therefore, a certain amount of urine moves from the lower end of the lower flow path of the paper strip guide 120 to the air pump 130 through the inside of the flow path 126 of the paper strip guide 120 due to a pressure difference, and the urine comes into contact and reacts with the paper strip 121 inserted into the paper strip guide 120 during movement.

In the urine test kit according to an embodiment of the present disclosure, the air pump 130 is used to enable the urine 10 contained in the container 110 to flow to the paper strip 121. Therefore, an examiner can perform a urine test at any desired time after urine collection, and the reliability of the urine test can be improved.

**FIG. 6A** to **FIG. 6G** are schematic diagrams for explaining various arrangements of grooves formed in the paper strip guide according to an embodiment of the present disclosure.

As described above with reference to **FIG. 2****,** the plurality of grooves 125 of the paper strip guide 120 may be formed in the lower portion of the paper strip guide 120 and merged into one groove in the upper portion of the paper strip guide 120. Therefore, the paper strip insertion hole 123 may be formed in each of the plurality of grooves 125 in the lower portion of the paper strip guide 120. Also, the paper strip insertion hole 123 may be spaced apart by a predetermined distance from the lower end of each of the plurality of grooves 125 formed in the lower portion of the paper strip guide 120.

As shown in **FIG. 6A****,** the paper strip guide 120 may include lower grooves 125 formed to have a maximum length in a longitudinal direction and upper grooves 125 formed to have a minimum length. That is, the upper grooves 125 may be formed to have a short length just enough to merge the plurality of grooves 125 formed in the lower portion of the paper strip guide 120 into one groove.

As shown in **FIG. 6B****,** an outer periphery of the paper strip guide 120 may be spaced apart by a predetermined distance from a region in which the grooves 125 are formed. Herein, if the paper strip guide 120 is attached to the container 110, the outer periphery of the paper strip guide 120 may be spaced apart by an appropriate distance from a region in which the grooves 125 are formed so that urine can flow to the paper strip through the flow path 126 formed in the paper strip guide 120.

Also, as shown in **FIG. 6C****,** the paper strip guide 120 may include the lower grooves 125 and the upper grooves 125 having a length equal to or greater than the lower grooves 125.

Further, as shown in **FIG. 6D****,** the paper strip guide 120 may include the lower grooves 125 which are formed linearly from a portion in which the paper strip insertion holes 123 are formed to a portion in which the upper grooves 125 are formed.

Besides, the paper strip guide 120 may be implemented in various forms as shown in **FIG. 6E** to **FIG. 6G****.**

The above description of the present disclosure is provided for the purpose of illustration, and it would be understood by a person with ordinary skill in the art that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A urine test kit, comprising:
a container configured to contain urine;
a paper strip guide including a groove and a paper strip which is formed in the groove and whose color is changed by the urine; and
an air pump configured to enable the urine to flow to the paper strip,
wherein the paper strip guide is attached to one surface of the container to form a flow path between the groove in the paper strip guide and the one surface of the container.

2. The urine test kit of Claim 1,
wherein the air pump is configured to enable the urine to flow to the paper strip through the flow path when a pressure difference is made between the inside and the outside of the air pump through a pumping operation by compressing and decompressing the air pump.

3. The urine test kit of Claim 1,
wherein the paper strip is spaced apart by a predetermined distance from a lower end of a lower flow path of the paper strip guide.

4. The urine test kit of Claim 1,
wherein the paper strip guide is spaced apart by a predetermined distance from a lower portion of the container and attached along an inner circumferential surface of the container.

5. The urine test kit of Claim 1, further comprising:
a lid configured to seal an upper portion of the container,
wherein the lid includes a lid connection hole in its upper surface, and a connection hose which connects the air pump and the paper strip guide passes through the lid connection hole.

6. The urine test kit of Claim 1,
wherein a container connection hole is formed in a side surface of the container, and a connection hose which connects the air pump and the paper strip guide passes through the container connection hole.

7. The urine test kit of Claim 1,
wherein a plurality of grooves is formed in a lower portion of the paper strip guide and merged into one groove in an upper portion of the paper strip guide.

8. A urine test kit, comprising:
a container configured to contain a liquid specimen;
a sheet which is attached to one surface of the container and in which a liquid specimen flow path is formed and at least one paper strip whose color is changed by the liquid specimen is provided in at least a part of the flow path; and
a pressing member configured to enable the liquid specimen to flow to the paper strip.

9. The urine test kit of Claim 8,
wherein when the pressing member is compressed, air inside the pressing member is discharged to the outside, and when the pressing member is decompressed, the liquid specimen flows to the paper strip through the flow path due to a pressure difference between the inside and the outside of the pressing member.
